(19) Europäisches Patentamt

European Patent Office

Office européen des brevets

(11) **EP 4 765 556 A1**

(12) **EUROPEAN PATENT APPLICATION**

(43) Date of publication:
24.06.2026 Bulletin 2026/26

(21) Application number: 24222093.7

(22) Date of filing: **20.12.2024**

(51) International Patent Classification (IPC):
$H02J\ 7/00^{(2026.01)}$ $H02J\ 50/12^{(2016.01)}$

(52) Cooperative Patent Classification (CPC):
**H02J 50/12; H02J 7/64;** H02J 2105/46

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Stichting IMEC Nederland**
**5656 AE Eindhoven (NL)**

(72) Inventor: **PASTERKAMP, Jesse**
**5616 SC Eindhoven (NL)**

(74) Representative: **AWA Sweden AB**
**Matrosgatan 1**
**Box 5117**
**200 71 Malmö (SE)**

(54) **A RECEIVER CIRCUITRY AND A METHOD FOR RECEIVING POWER THROUGH WIRELESS POWER TRANSFER, AND AN IMPLANT DEVICE**

(57)  A receiver circuitry (100) for receiving power through wireless power transfer comprises: a power receiver (110) comprising a resonant circuit (112) and a first switch (126), and configured to store received energy through resonance, wherein the first switch (126) is in a closed state for build-up of energy in the resonant circuit (112) and in an open state for forcing output of a current from an output node (118); a second switch (128) configured in an open state to disconnect the output node (118) from a load (130) and in a closed state connect the output node (118) to the load (130); and a control unit (150) configured to control states of the first and second switches (126, 128) based on a relation of a number of resonance cycles to a cycle threshold value and on a relation of an amount of energy built up to an energy threshold value.

Fig. 1

EP 4 765 556 A1

# Description

Technical field

The present description relates to a receiver circuitry and a method for receiving power through wireless power transfer. The present description further relates to an implant device comprising the receiver circuitry.

Background

Power transfer may be used in various applications in order to provide power to a receiver circuitry such that a device in which the receiver circuitry is arranged remains operational.

Wireless powering by means of wireless powering is useful in numerous applications. By using wireless powering, a wire between a powering apparatus and a receiver circuitry is not needed so as to provide a convenient or practical manner of powering the receiver circuitry. Also, in some instances, it is not possible to use a wire for powering, such as when the receiver circuitry is part of a device arranged in a body of a person as an implanted or ingested device. Then, wireless powering can be used in order to enable a prolonged lifetime or act as a primary source of power of the implanted or ingested device.

Implant devices with small form factors are increasingly used for various purposes. The implant devices may further have a dynamic load requiring relatively high power at one point in time and low power at another point in time. Thus, there is a need for a small wireless power receiver circuitry that may be efficiently used at relatively high power levels while supporting also low power levels with high efficiency.

Summary

An objective of the present description is to provide an efficient power transfer to a receiver circuitry through wireless power transfer. Another objective of the present description is to provide efficient power transfer to a small receiver circuitry.

These and other objectives are at least partly met by the invention as defined in the independent claims. Preferred embodiments are set out in the dependent claims.

According to a first aspect, there is provided a receiver circuitry for receiving power through wireless power transfer, said receiver circuitry comprising: a power receiver comprising a resonant circuit and a first switch, wherein the power receiver is configured to receive an alternating current, AC, wireless power signal and configured to store energy through resonance, wherein the first switch is configured to be controlled between a closed state, wherein the resonant circuit is closed for build-up of energy in the resonant circuit, and an open state, wherein the resonant circuit is open for forcing output of a current from an output node of the power receiver; a second switch configured to be controlled between an open state for disconnecting the output node of the power receiver from a load and a closed state for connecting the output node of the power receiver to the load, wherein the second switch is configured to be set in an open state during build-up of energy in the resonant circuit; and a control unit configured to control a state of the first switch and a state of the second switch, wherein the control unit is configured to set the state of the first switch and set the state of the second switch based on a relation of a number of resonance cycles to a cycle threshold value and based on a relation of an amount of energy built up in the power receiver to an energy threshold value.

The receiver circuitry according to the first aspect is configured to allow the resonant circuit to resonate for a number of cycles. When the first switch is opened, current flow in the resonant circuit is opened forcing current to be output from the output node of the power receiver through the second switch to the load.

The receiver circuitry according to the first aspect may be referred to as a current-mode (CM) rectifier. In comparison to a voltage-mode rectifier, the receiver circuitry does not need a separate direct current (DC) to direct current converter. Rather, the CM rectifier may regulate an output voltage to a suitable DC supply directly. Thus, the receiver circuitry may be very compact and is suitable for use in small form-factor devices. Hence, the receiver circuitry may be suitable for use in an implanted device which may be required to be very small.

The control unit is configured to control states of the first and second switches based on the number of resonance cycles. This implies that the receiver circuitry can be configured to operate using a number of resonance cycles for efficient charging. The number of resonance cycles to be used may have an optimum that is related to a design of the resonant circuit. The receiver circuitry may thus be configured to control the first and second switches such that the resonant circuit is allowed to resonate a number of cycles that correspond to or is at least close to an optimum for the receiver circuitry.

Thanks to the control unit being further configured to control the states of the first and second switches based on an amount of energy being built up in the power receiver, the receiver circuitry may further ensure that power is efficiently received by the receiver circuitry. In particular, if a relatively high power is received from a powering apparatus, a large amount of energy may be built up in the resonant circuit during the allowed number of resonance cycles. This implies that, when the output of the current is forced from the output node of the power receiver, energy may be lost due to a too high energy being output. This is avoided thanks to the control unit controlling the states of the first and second switches based on the relation of the amount of energy built up to the energy threshold value.

For instance, the circuitry may comprise an overvoltage protection component to ensure that a threshold voltage is not exceeded. Similarly, the circuitry may be configured to impose a limitation on the current to ensure that a maximum current density of a device is not exceeded. Thus, if the receiver circuitry outputs a too high energy, energy may be lost in a restricting component or the device may be damaged.

Thus, the receiver circuitry is configured to ensure that energy is not lost in restricting components and safety limits of the components are respected. This implies that the energy received by the receiver circuitry can be efficiently provided to the load.

The receiver circuitry may be advantageously used in a device that has a high power consumption and therefore a need for receiving relatively high power. For instance, some implant devices may have features requiring high power consumption, such as implant devices being used for providing stimulation signals to a body in which the implant device is implanted. This may for instance be stimulation signals for stimulating a nerve. Thus, the receiver circuitry may be configured to receive relatively high power through the wireless power consumption. Thanks to the use of the receiver circuitry, energy losses in restricting components may be avoided.

It should be realized that the term receiver circuitry is used for referring to the circuitry that receives power through wireless power transfer but also provides the received power to a load. The receiver circuitry may be connected to a battery at the load and may thus form a charging circuitry. However, the receiver circuitry may also or alternatively act as a primary source of power to the load.

The receiver circuitry includes a power receiver, which forms the part of the receiver circuitry wherein power is received through wireless power transfer.

The power receiver may comprise a resonant circuit defining a resonant frequency. For instance, the power receiver may comprise an inductor (L) and a capacitor (C), defining an LC circuit having a resonant frequency defined by the inductance and capacitance of the inductor and the capacitor, respectively. The resonant circuit may be configured to build up energy based on resonance at a frequency corresponding to the resonant frequency of the power receiver. An amplitude of a signal at a node of the resonant circuit may increase for each cycle of the resonant frequency as long as energy is stored and not extracted from the power receiver. A resonance cycle corresponds to a single period of a signal at the resonant frequency.

The power receiver may be configured to receive power through inductive wireless power transfer. However, it should be realized that the power receiver may store energy through resonance in another manner such that the wireless power transfer need not necessarily be performed by inductive wireless power transfer. For instance, the power receiver may alternatively be configured to receive power through capacitive resonant wireless power transfer.

Each of the first switch and the second switch may be any type of switch that allows control of connecting or disconnecting two nodes to each other. Thus, the first switch may be configured to control connection or disconnection of two nodes in the resonant circuit. The second switch may be configured to control connection or disconnection of two nodes between the output node of the power receiver and the load. As an example, each of the first switch and the second switch may be a transistor which may be controlled by a control signal applied to a gate of the transistor for opening or closing a connection between the two nodes.

The load may be any circuitry configured to be connected to an output of the receiver circuitry for receiving power from the receiver circuitry.

The control unit may be any unit which may be configured to control states of the switches. The control unit may be a logic unit which may be configured to provide an output in dependence of input signals. The logic unit may be implemented in hardware, providing for a very fast response, and ensuring that the control unit may be provided in small size. The control unit may be configured to provide control signals to the first and second switches based on analog input signals. However, it should be realized that the control unit may alternatively be configured as a processing unit, which may for instance be configured to process digital signals for controlling the states of the switches.

The amount of energy built up in the power receiver may for instance be represented by a current or a voltage. The built-up energy may be compared to a corresponding energy threshold value. Thus, the energy threshold value may in some embodiments be provided as a threshold voltage and may in other embodiments be provided as a threshold current.

A restriction component of the receiver circuitry may be configured to control an output voltage to be provided to the load. However, in some scenarios, the restriction component may instead be configured to control an output current to be provided to the load. In such case, the second switch need not necessarily be controlled and may instead be provided as a diode.

In other words, according to a separate aspect, there is provided a receiver circuitry for receiving power through wireless power transfer, said receiver circuitry comprising: a power receiver comprising a resonant circuit and a first switch, wherein the power receiver is configured to receive an alternating current, AC, wireless power signal and configured to store energy through resonance, wherein the first switch is configured to be controlled between a closed state, wherein the resonant circuit is closed for build-up of energy in the resonant circuit, and an open state, wherein the resonant circuit is open for forcing output of a current from an output node of the power receiver; a control component configured to control current between the output node of the power receiver and a load; and a control unit configured to control a state of the first switch, wherein the control unit is configured to set the state of the first switch based on a relation of a number of resonance cycles to a cycle threshold value and based on a relation of an amount of energy built up in the power receiver to an energy threshold value.

The control component may be a static diode. However, as described above in the receiver circuitry of the first aspect, the control component may be a second switch and, in such case, the control unit is further configured to set the state of the second switch as described above.

According to an embodiment, the receiver circuitry further comprises an overvoltage protection component connected to the output node of the power receiver, wherein the control unit is configured to set the state of the first switch and set the state of the second switch based on the relation of the amount of energy built up in the power receiver to the energy threshold value, wherein the energy threshold value is a threshold voltage for overvoltage protection.

The receiver circuitry may thus be configured to monitor a voltage representing the amount of energy built up in the power receiver in relation to a threshold voltage for overvoltage protection. This implies that the control unit may be configured to control output of current form the output node of the power receiver such that losses in the overvoltage protection component are avoided or such that losses in the overvoltage protection component are reduced.

Thus, when the threshold voltage is exceeded, the control unit may be configured to force output of current from the power receiver to avoid further build-up of energy in the resonant circuit that may anyway be lost in the overvoltage protection component. This ensures that the receiver circuitry may efficiently use the power received through wireless power transfer.

The overvoltage protection component may be implemented as an overvoltage protection diode. However, it should be realized that the overvoltage protection component may be implemented in other ways. For example, a gas discharge tube may be used, or a metal-oxide-semiconductor (MOS)-based clamp may be used.

According to an embodiment, the control unit is configured to determine, during storing of energy through resonance by the power receiver, whether a first condition or a second condition is met, wherein the control unit is further configured to, upon determining that the first condition or the second condition is met, control switching of the state of the first switch to the open state and the state of the second switch to the closed state.

Thus, the control unit may be configured to determine two conditions for controlling the switching of the first and second switches. This is a suitable manner for providing control of the first switch and the second switch based on the relation of number of resonance cycles to the cycle threshold value and based on the relation of the amount of energy built up in the power receiver to the energy threshold value.

This facilitates providing a control for allowing the resonant circuit to resonate during a number of cycles corresponding to at least close to the optimum, as long as the energy threshold value is not exceeded. Thus, the receiver circuitry provides efficient charging through wireless power transfer.

According to an embodiment, the second condition is based on the relation of the amount of energy built up in the power receiver to the energy threshold value, wherein the control unit is configured to determine that the second condition is met based on receiving input that the energy threshold value is exceeded.

Thus, the control unit may be configured to receive an input signal indicating that the energy threshold value is exceeded by the amount of energy being built up in the power receiver. Hence, the control unit may be configured to control the first switch and the second switch based on this input signal such that the resonant circuit may be opened for forcing output of the current from the output node of the power receiver when the energy threshold value is exceeded.

According to an embodiment, the first condition is based on the relation of the number of resonance cycles to the cycle threshold value, wherein the control unit is configured to determine that the first condition is met by determining that the number of resonance cycles exceeds the cycle threshold value.

Hence, the control unit may be configured to control the first switch and the second switch based on determination that the number of resonance cycles exceeds the cycle threshold value such that the resonant circuit may be opened for forcing output of the current from the output node of the power receiver when the cycle threshold value is exceeded. This may ensure that the current may be output from the power receiver when a number of resonance cycles corresponds to or is at least close to an optimum number of cycles.

According to an embodiment, the receiver circuitry further comprises a comparator, wherein the comparator is connected to a node of the power receiver for comparing the amount of energy built up in the power receiver to the energy threshold value, wherein the comparator is configured to provide input to the control unit in dependence of the comparing of the amount of energy built up in the power receiver to the energy threshold value.

Thus, the comparator may be used for determining whether the second condition is met. This is a simple manner for making the determination, which may be provided by a small component, such that the receiver circuitry may be compact.

The comparator may be connected to a suitable node of the power receiver. For instance, the comparator may be connected to a comparator node opposite to the output node of the power receiver. Thus, an inductor of the resonant circuit may be arranged between the output node and the comparator node. Similarly, a capacitor of the resonant circuit may also be arranged between the output node and the comparator node, the inductor and the capacitor being connected in parallel in the resonant circuit.

According to an embodiment, the comparator is configured to compare a power receiver voltage at the node of the power receiver to a threshold voltage during a negative part of the power receiver voltage for providing input to the control unit for switching the state of the first switch and the state of the second switch during a following positive part of the power receiver voltage.

The output node of the power receiver may be connected to a positive part of components in the resonant circuit, such as being connected to a positive part of the inductor. Thus, the output of the current from the output node should be performed at a positive part of the power receiver voltage.

Hence, by comparing the power receiver voltage to the threshold voltage during a negative part of the power receiver voltage, a determination whether the second condition is met may be performed close in time to outputting of the current from the output node. This may ensure that the output may be performed very soon after a determination that the second condition is met. This is done to ensure little to no time is left for energy in the resonant element to increase and exceed device constraints between the determination of the comparator and the time that current is output from the output node.

According to an embodiment, further comprising a counter unit configured to count a number of resonance cycles of the resonant circuit during build-up of energy.

Thus, the counter unit may be configured to determine the number of resonance cycles during which energy is allowed to build up in the power receiver. The counter unit may thus provide a value to be compared to the cycle threshold value. This is a suitable manner for providing input for determining whether the first condition is met.

However, it should be realized that a delay corresponding to the first condition being met may be determined in other manners. The delay is set by the number of resonance cycles corresponding to the cycle threshold value. Since a time period of this delay may be known and correspond to a number of periods of the resonant frequency, a delay circuit may be used. The delay circuit may provide an output that goes high (or low) when a desired time period has passed.

According to an embodiment, the control unit is configured to receive the cycle threshold value, wherein the cycle threshold value is based on a quality factor of the resonant circuit.

Thus, the control unit may be configured to determine a relation between the number of resonance cycles and the cycle threshold value being available to the control unit.

The cycle threshold value may be based on the quality factor of the resonant circuit. This may be a suitable manner of defining an optimum number of resonance cycles during which energy should be allowed to build up in the power receiver.

According to an embodiment, the cycle threshold value is set to the quality factor divided by 2.51. This provides a value defining the optimum number of resonance cycles during which energy should be allowed to build up in the power receiver.

According to an embodiment, the second switch comprises a transistor, wherein the second switch is configured to be controlled between the open state and the closed state based on a signal to a gate of the transistor.

This provides a suitable manner for controlling a connection or disconnection to be provided by the second switch. The transistor may be easily controlled by controlling the signal to the gate. Thus, the control unit may be configured to output a control signal to the second switch, wherein the control signal is provided to the gate of the transistor.

According to an embodiment, the control unit is configured to set the state of the first switch to the open state in dependence of a voltage of the load reaching a target level.

This implies that the resonant circuit may be opened when there is no need for receiving more power at the load. Thus, in order to avoid energy to be lost in the resonant circuit, e.g., as power dissipated in the inductor, when no further charging of outputs of the receiver circuitry is needed, the resonant circuit may be opened.

The receiver circuitry may be configured to receive input from a load regulation comparator for determining when the voltage of the load has reached the target level.

According to a second aspect, there is provided an implant device comprising the receiver circuitry according to the first aspect, wherein the implant device is configured to be implanted in a body and configured to receive power through wireless power transfer to the receiver circuitry.

Effects and features of this second aspect are largely analogous to those described above in connection with the first aspect. Embodiments mentioned in relation to the second aspect are largely compatible with the first aspect.

The receiver circuitry may be advantageously used in applications wherein a small form factor is needed, which may typically be needed in an implant device. Further, when powering the implant device, efficient use of the power may be desired as a coupling between a powering apparatus and the receiver circuitry of the implant device may be weak as the wireless power transfer may need to be made through tissue while a signal strength may need to be limited in order not to cause any damage to tissue through which powering signals are transmitted.

Thus, thanks to the receiver circuitry of the first aspect being configured to efficiently use received power, the receiver circuitry of the first aspect may be particularly suitable for use in the implant device.

According to a third aspect, there is provided a method for receiving power through wireless power transfer, said method comprising: receiving, by a power receiver comprising a resonant circuit, an alternating current (AC) wireless power signal and storing energy through resonance by a first switch in the resonant circuit being closed for build-up of energy in the resonant circuit; determining a first condition based on a relation of a number of resonance cycles to a cycle threshold value and determining a second condition based on a relation of an amount of energy built up in the power receiver to an energy threshold value; upon determining that the first condition or the second condition is met, controlling switching of the first switch to an open state for forcing output of a current from an output node of the power receiver, and controlling switching of a second switch to a closed state for connecting the power receiver to a load.

Effects and features of this third aspect are largely analogous to those described above in connection with the first and second aspects. Embodiments mentioned in relation to the third aspect are largely compatible with the first and second aspects.

Thanks to the controlling of the states of the first and second switches being based on both a number of resonance cycles and on an amount of energy being built up in the power receiver, the method may ensure that power is efficiently received.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

    Fig. 1 is a schematic view of a receiver circuitry according to an embodiment.
    Fig. 2 is a schematic view of a receiver circuitry according to another embodiment.
    Fig. 3 is a schematic view of an implant device according to an embodiment.
    Fig. 4 is a flow chart of a method according to an embodiment.

Detailed description

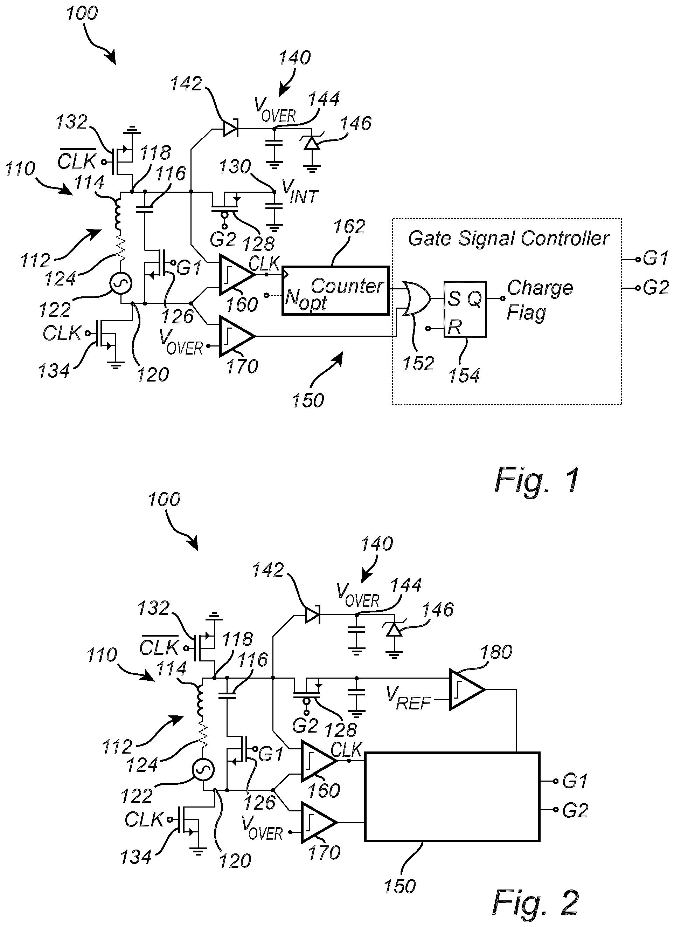

Referring now to Fig. 1, a receiver circuitry 100 according to an embodiment will be described. The receiver circuitry 100 comprises a power receiver 110. The power receiver 110 shown in Fig. 1 comprises a resonant circuit 112 having an inductor 114 connected in parallel with a capacitor 116 between a first node 118 arranged at a positive side of the inductor 114 and the capacitor 116 and a second node 120 arranged at a negative side of the inductor 114 and the capacitor 116. The inductor 114 and the capacitor 116 form an LC-tank configured to receive an alternating current (AC) power signal through resonant inductive wireless power transfer. However, it should be realized that the power receiver 110 may be configured in a different manner for receiving the AC wireless power signal. For instance, the power receiver 110 may be configured for capacitive resonant wireless power transfer.

The power receiver 110 is further illustrated as comprising a voltage source 122. This is illustrated as representing the power received through wireless power transfer to the power receiver 110. Thus, the voltage source 122 is provided by the voltage induced by the inductor 114 receiving an inductive wireless AC signal. The power receiver 110 is also illustrated as comprising a resistor 124 connected in series to the inductor 114. The resistor 124 represents a resistance in the power receiver 110.

The resonant circuit 112 defines a resonant frequency based on the inductance and capacitance of the inductor 114 and the capacitor 116, respectively. The resonant circuit 112 may be configured to build up energy based on resonance at a frequency corresponding to the resonant frequency of the resonant circuit 112.

The power receiver 110 further comprises a first switch 126. The first switch 126 is arranged in the resonant circuit 112 for selectively closing or opening the resonant circuit 112. The first switch 126 may be arranged at a negative side of the capacitor 116 between the capacitor 116 and the second node 120.

The first switch 126 may be controlled between a closed state and an open state. In the closed state, the first switch 126 connects the capacitor 116 to the second node 120 such that the resonant circuit 112 is closed and allowed to build up energy. In the open state, the first switch 126 disconnects the capacitor 116 from the second node 120, wherein the resonant circuit 112 is open such that further energy is not built up in the resonant circuit 112.

The power receiver 110 is further configured to output energy to a load. The power receiver 110 thus comprises an output node at which energy may be output from the power receiver 110. The output node may be the first node 118 and the first node is further referred to herein as the output node 118 of the power receiver 110.

The receiver circuitry 100 further comprises a second switch 128. The second switch 128 is connected to output node 118 of the power receiver 110. The second switch 128 is configured to be controlled between an open state for disconnecting the output node 118 of the power receiver 110 from a load 130 and a closed state for connecting the output node 118 of the power receiver 110 to the load 130.

When power is received by the power receiver 110, the first switch 126 is set to the closed state for allowing build-up of energy in the resonant circuit 112. Also, the second switch is set to the open state during build-up of energy in the resonant circuit 112 such that the resonant circuit 112 is isolated from the load 130 allowing build-up of energy.

An amplitude of a signal at the output node 118 of the power receiver 110 may increase for each cycle of the resonant frequency as long as energy is stored and not extracted from the power receiver 110. A resonance cycle corresponds to a single period of a signal at the resonant frequency.

The first node 118 of the power receiver 110 may be connected to ground via a first ground connection switch 132. Similarly, the second node 120 of the power receiver 110 may be connected to ground via a second ground connection switch 134. The first and second ground connection switches 132, 134 may each be formed by a respective transistor, which may be controlled between an open state and a closed state based on a signal received at a gate of the respective transistor.

The second ground connection switch 134 may be configured to receive a clock signal at the gate, while the first ground connection switch 132 may be configured to receive an inverted clock signal at the gate. Thus, the first node 118 and the second node 120 are alternatingly connected to ground at a frequency set by the clock signal. The clock signal may be provided at a frequency corresponding to the resonant frequency of the resonant circuit 112 and in phase with a signal resonating in the resonant circuit 112 for building up energy in the resonant circuit 112.

When the power receiver 110 outputs energy to the load 130, the first switch 126 is set to the open state to interrupt current flow in the resonant circuit 112 for forcing output of a current from the output node 118 of the power receiver 110. Further, the second switch 128 is set to the closed state for connecting the output node 118 to the load 130.

The power receiver 110 may be referred to as a current-mode rectifier.

The resonant circuit 112 may be allowed to resonate for building up energy in the resonant circuit 112 during a number of cycles. The number of cycles during which the resonant circuit 112 resonates before the current is output from the output node 118 may be referred to as $N_{reso}$.

In order to achieve efficient operation, the current-mode rectifier should operate such that $N_{reso}$ is equal to or close to an optimal number of cycles, $N_{opt}$. The optimal number of cycles may be determined by finding a number of cycles N for which:

$$\frac{dV_{LC}(N)}{dN} = 0,$$

where $V_{LC}(N)$ is the amplitude of a sinusoidal waveform of the voltage at the output node 118 after $N$ cycles. The voltage signal $v_{LC}$ at the output node 118 may be defined as $v_{LC} = v_{LC} \sin 2\pi ft$, with $N = tf$, and $t$ being time and $f$ being the resonance frequency of the system.

This may equivalently be expressed as:

$$N_{opt} = argmax_N \left\{ \frac{V_{LC}(N)}{N} \right\}.$$

An analysis of the optimal number of cycles may give the optimal number as $N_{opt} = Q/2.51$, where $Q$ is a quality factor of the LC-tank.

Energy may be lost in the receiver circuitry, if the energy output at the output node 118 from the power receiver 110 exceeds limitations set by a restriction component. Therefore, in order to ensure that power is efficiently received by the receiver circuitry 100, the receiver circuitry 100 is further configured to avoid building up energy exceeding limitations set by the restriction component.

In particular, the receiver circuitry 100 may comprise an overvoltage protection component 140. The overvoltage protection component 140 may be configured to define a threshold voltage. If the output from the output node 118 exceeds the threshold voltage, the output may be clipped, and energy lost in the overvoltage protection component 140.

The overvoltage protection component 140 may be designed such that a forward voltage of the Zener diode 146 is smaller than that of the first diode 142, meaning the first diode 142 is protected from large currents which subsequently also protects other components at the load 130 from injected currents. Thus, a substrate of an application-specific integrated circuit (ASIC) which is to be powered by the receiver circuitry 100 may be protected from injected currents. The overvoltage protection component 140 provides a passive protection to ensure safety.

The overvoltage protection component 140 may further function as an electrostatic discharge protection.

It should be realized that the overvoltage protection component 140 may be implemented in other ways as would be understood by a person skilled in the art.

The receiver circuitry 100 may be configured to control the resonant circuit 112 such that the number of cycles during which the resonant circuit 112 is allowed to resonate may be defined based on two conditions. Thus, according to a first condition, the number of cycles may be determined as:

$$N_{opt} = \frac{Q}{2.51}.$$

This condition is used when the output is not limited by a threshold voltage $V_{over}$ of the overvoltage

protection component 140. In other words, this condition is applied when the voltage at the output node 118 is

$$V_{LC}(N = Q/2.51) < V_{over}.$$

According to a second condition, the number of cycles may be determined as:

$$N_{opt} = N \text{ where } V_{LC}(N) = V_{over}.$$

This condition is used when the voltage at the output node 118 exceeds the threshold voltage $V_{over}$ before the number of cycles for the first condition have been reached.

Thanks to such control, the receiver circuitry 100 ensures that power is efficiently received.

The receiver circuitry 100 comprises a control unit 150 which is configured to provide control of the energy being built up in the power receiver 110 for ensuring an efficient receipt of power. The control unit 150 is configured to set the state of the first switch 126 and set the state of the second switch 128 based on a relation of a number of resonance cycles to a cycle threshold value and based on a relation of an amount of energy built up in the power receiver to an energy threshold value.

In the embodiment shown in Fig. 1, the energy threshold value is set by the threshold voltage $V_{over}$ of the overvoltage protection component 140. Thus, the control unit 150 is configured to set the state of the first switch 126 and set the state of the second switch 128 based on the relation of the amount of energy built up in the power receiver 110 to the threshold voltage for overvoltage protection component 140.

The control unit 150 may be configured to determine, during storing of energy through resonance by the power receiver 110, whether the first condition or the second condition is met. In particular, the control unit 150 may be configured to determine whether the first condition or the second condition as defined above are met.

The control unit 150 is further configured to, upon determining that the first condition or the second condition is met, control switching of the state of the first switch 126 to the open state and the state of the second switch 128 to the closed state. Thus, the control unit 150 is configured to stop accumulation of energy in the resonant circuit 112 and force output of a current from the output node 118, when one of the first or the second condition is met.

The receiver circuitry 100 may comprise a first comparator 160 for identifying resonance cycles. The first comparator 160 may have two input ports connected to the output node 118 and the second node 120, respectively. The first comparator 160 may thus be configured to determine whether the signal at the output node 118 is larger than the signal at the second node 120. The first comparator 160 may provide an output signal that is high when the signal at the output node 118 is larger than the signal at the second node 120 and that is low when the signal at the output node 118 is smaller than the signal at the second node 120.

The receiver circuitry 100 may further comprise a counter unit 162 configured to receive the output signal output by the first comparator 160 as an input signal to the counter unit 162. The counter unit 162 may thus be configured to detect when the input signal (i.e., the output signal from the first comparator 160) goes high and to count the number of times the input signal goes high. The counter unit 162 is thus configured to count a number of resonance cycles of the resonant circuit during build-up of energy.

The counter unit 162 may form part of the control unit 150. The counter unit 162 may further be configured to receive a cycle threshold value which may define the number of cycles during which the resonant circuit 112 is allowed to resonate for building up energy in the resonant circuit 112. The cycle threshold value received by the counter unit 162 may be a desired number of cycles, $N_{opt}$, which may be determined as described above. The cycle threshold value may thus be based on a quality factor of the resonant circuit 112.

The counter unit 162 may thus be configured to compare a counted number of resonance cycles to the cycle threshold value. The counter unit 162 may provide an output that is dependent on whether the counted number of resonance cycles is larger than the cycle threshold value or not. Thus, when the counted number of resonance cycles exceeds the cycle threshold value, the output from the counter unit 162 may go high, which may be further used for controlling the states of the first switch 126 and the second switch 128.

It should be realized that the first comparator 160 and the counter unit 162 ensure that the output from the counter unit 162 going high is delayed for a period of time corresponding to the number of resonance cycles exceeding the cycle threshold value. It should be realized that the control unit 150 may provide a delay in different manners, such as including a delay line, comprising a sequence of delay elements.

The receiver circuitry 100 may further comprise a second comparator 170 for identifying when the amount of energy being built up in the power receiver 110 exceeds a limit. The second comparator 170 may be configured to determine whether the amount of energy being built up in the power receiver 110 exceeds a limit based on the threshold voltage for overvoltage protection forming the energy threshold value.

The second comparator 170 is connected to a node of the power receiver 110, such as the second node 120, for comparing the amount of energy built up in the power receiver 110 to the energy threshold value. The second comparator 170 may thus receive a signal at the second node 120 and the threshold voltage as inputs.

The second comparator 170 may provide an output signal that is high when the signal at the second node 120 is larger than the threshold voltage and that is low when the signal at the second node 120 is smaller than the threshold voltage. The output signal from the second comparator 170 may be provided as an input signal to the control unit 150.

The second comparator 170 may thus be configured to provide input to the control unit 150 in dependence of the comparing of the amount of energy built up in the power receiver to the energy threshold value. The control unit 150 may thus be configured to determine that the second condition is met based on receiving input that the energy threshold value is exceeded. The input for determining that the second condition is met may thus be received as the output signal from the second comparator 170.

It should be realized that the relation between the amount of energy built up in the power receiver 110 and the energy threshold value may be determined in other manners. For instance, the voltage at a node of the power receiver 110 may be combined in alternative manners to the threshold voltage for determining the relation therebetween.

It should be noted that, since the second comparator 170 is connected to the second node 120 arranged at the negative side of the inductor 114 and the capacitor 116, the second comparator 170 is configured to compare a negative part of a power receiver voltage to the threshold voltage. In other words, when the voltage at the second node 120 is positive, the voltage at the first node 118 at which output from the power receiver 110 is made, is negative. This implies that, when the voltage at the second node 120 exceeds the threshold voltage, the voltage at the output node 118 will be low, as the output node 118 is connected to ground via the a first ground connection switch 132.

Hence, the control unit 150 may further be configured to control switching of the states of the first switch 126 and the second switch 128 during a following positive part of the power receiver voltage at the output node 118 such that the current may be output from the output node 118 during a positive part following the negative part during which the exceeding of the threshold voltage was identified. This implies that the current may be output at a point in time close to detection that the threshold voltage is exceeded. This ensures that the amount of energy lost due to overvoltage protection may be low.

The output from the counter unit 162 may provide an indication whether the first condition is met and the output from the second comparator 170 may provide an indication whether the second condition is met.

The control unit 150 may comprise a digital logic gate that receives the output from the counter unit 162 and the output from the second comparator 170. The digital logic gate may be an OR gate 152, which may thus implement an OR function. The OR gate 152 may provide an output that is high if any of the inputs is high, i.e., if the first condition or the second condition is met.

The control unit 150 may further comprise a state controller 154 which may store states of control signal to be used for setting the states of the first switch 126 and the second switch 128. For instance, the control unit 150 may comprise a set-reset (SR) latch, which receives the output from the OR gate 152 and sets a sate of control signals accordingly. The output from the SR latch 154 may be configured to indicate a charge flag which sets the states of the control signals for providing charging from the receiver circuitry 100 to the load 130.

The first switch 126 may be formed by a transistor. This implies that a signal to a gate of the transistor may control whether the first switch 126 is in the open state or the closed state. Similarly, the second switch 128 may be formed by a transistor. This implies that a signal to a gate of the transistor may control whether the first switch 128 is in the open state or the closed state.

The control unit 150 may further be configured to output control signals to the gates of the first switch 126 and the second switch 128. The control unit 150 may be configured, when the charge flag is provided by the SR latch 154, to set the control signals on a rising edge of the clock signal. The control signal G1 to the gate of the first switch 126 may be high when energy is built up in the resonant circuit 112 and may turn low upon the output from the OR gate 152 going high for forcing output of the current from the output node 118. The control signal G2 to the gate of the second switch 128 may be low when energy is built up in the resonant circuit 112 and may turn high upon the output from the OR gate 152 going high for connecting the output node 118 to the load 130.

Referring now to Fig. 2, the receiver circuitry 100 according to another embodiment is discussed.

The receiver circuitry 100 shown in Fig. 2 may be identical to the receiver circuitry discussed above in relation to Fig. 1, with an addition of a load regulation comparator 180.

A load regulation comparator 180 may be applied to each output of the receiver circuitry 100, one load regulation comparator 180 per output. Although not shown in Fig. 2, there may also be a load regulation comparator monitoring the threshold voltage at the overvoltage protection component 140.

The load regulation comparator 180 compares an output voltage $V_{INT}$ provided to the load 130 to a reference voltage $V_{ref}$. When the output voltage exceeds the reference volage (or a voltage derived from the output voltage exceeds the reference voltage), an output from the load regulation comparator 180 goes high.

The control unit 150 may be configured to receive the output from the load regulation comparator 180. If all outputs of the receiver circuitry 100 are charged fully such that all load regulation comparators 180 give a high output, the control unit 150 may control the first switch 126 to be set to the open state. Thus, the control unit 150 may be configured to set the state of the first switch 126 to the open state in dependence of a voltage of the load 130 reaching a target level.

The control unit 150 will thus set the first switch 126 to the open state such that the power receiver 110 is controlled to stop the receiving of power. When one load drops below the target level again, the control unit 150 may again control the state of the first switch 126 to the closed state, such that the power receiver 110 is turned on and the receiving of power is resumed.

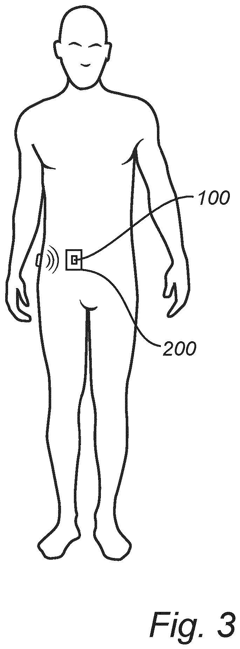

Referring now to Fig. 3, the receiver circuitry 100 according to any of the above-described embodiments may be advantageously provided in an implant device 200. Although it should be realized that the receiver circuitry 100 may be part of any device for use in any wireless power transfer regardless of where the device to be powered is arranged in relation to a power transmitter, the receiver circuitry 100 is particularly suited for use in the implant device 200. The implant device 200 may be configured to be implanted in a human body or an animal body and may need wireless power transfer for powering the implant device 200 while being implanted.

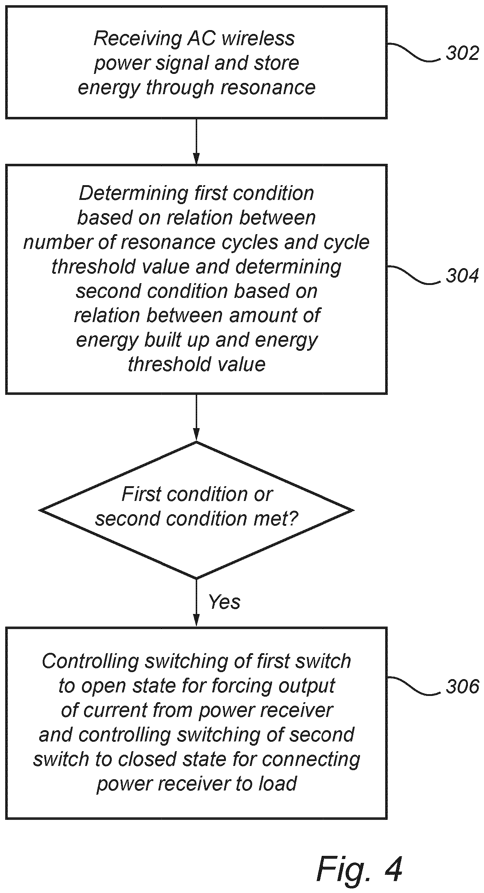

Referring now to Fig. 4, a method for receiving power through wireless power transfer will be described.

The method comprises receiving 302, by a power receiver comprising a resonant circuit, an AC wireless power signal and storing energy through resonance by a first switch in the resonant circuit being closed for build-up of energy in the resonant circuit.

Further, during build-up of energy in the resonant circuit, a second switch which is configured to control a connection between an output node of the power receiver and a load may be set to an open state. The second switch may thus be configured to isolate the resonant circuit from the load for allowing build-up of energy in the resonant circuit.

The method further comprises determining 304 a first condition based on a relation of a number of resonance cycles to a cycle threshold value and determining a second condition based on a relation of an amount of energy built up in the power receiver to an energy threshold value.

Thus, the method may involve checking two conditions for controlling a period during which the resonant circuit is allowed to build up energy. The method may thus ensure that the resonant circuit is allowed to build up energy using a number of resonance cycles for efficient charging. However, the method may further ensure that the amount of energy built up in the power receiver can be terminated when the amount of energy exceeds an energy threshold value such that energy will not be unnecessarily lost, e.g., in an overvoltage protection component, when the energy is output from the power receiver. Thus, if the amount of energy exceeds the energy threshold value before a desired number of resonance cycles have passed, the building of energy may be stopped.

The method further comprises, upon determining that the first condition or the second condition is met, controlling switching 306 of the first switch to an open state for forcing output of a current from the output node of the power receiver, and controlling switching of a second switch to a closed state for connecting the power receiver to a load.

This implies that the method ensures that power is efficiently received.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A receiver circuitry (100) for receiving power through wireless power transfer, said receiver circuitry (100) comprising:

   a power receiver (110) comprising a resonant circuit (112) and a first switch (126), wherein the power receiver (110) is configured to receive an alternating current, AC, wireless power signal and configured to store energy through resonance, wherein the first switch (126) is configured to be controlled between a closed state, wherein the resonant circuit (112) is closed for build-up of energy in the resonant circuit (112), and an open state, wherein the resonant circuit (112) is open for forcing output of a current from an output node (118) of the power receiver (110); a second switch (128) configured to be controlled between an open state for disconnecting the output node (118) of the power receiver (110) from a load (130) and a closed state for connecting the output node (118) of the power receiver (118) to the load (130), wherein the second switch (128) is configured to be set in an open state during build-up of energy in the resonant circuit (112); and a control unit (150) configured to control a state of the first switch (126) and a state of the second switch (128), wherein the control unit (150) is configured to set the state of the first switch (126) and set the state of the second switch (128) based on a relation of a number of resonance cycles to a cycle threshold value and based on a relation of an amount of energy built up in the power receiver (110) to an energy threshold value.

2. The receiver circuitry according to claim 1, further comprising an overvoltage protection component (140) connected to the output node (118) of the power receiver (110), wherein the control unit (150) is configured to set the state of the first switch (126) and set the state of the second switch (128) based on the relation of the amount of energy built up in the power receiver (110) to the energy threshold value, wherein the energy threshold value is a threshold voltage for overvoltage protection.

3. The receiver circuitry according to claim 1 or 2, wherein the control unit (150) is configured to determine, during storing of energy through resonance by the power receiver (110), whether a first condition or a second condition is met, wherein the control unit (150) is further configured to, upon determining that the first condition or the second condition is met, control switching of the state of the first switch (126) to the open state and the state of the second switch (128) to the closed state.

4. The receiver circuitry according to claim 3, wherein the second condition is based on the relation of the amount of energy built up in the power receiver (110) to the energy threshold value, wherein the control unit (150) is configured to determine that the second condition is met based on receiving input that the energy threshold value is exceeded.

5. The receiver circuitry according to claim 3 or 4, wherein the first condition is based on the relation of the number of resonance cycles to the cycle threshold value, wherein the control unit (150) is configured to determine that the first condition is met by determining that the number of resonance cycles exceeds the cycle threshold value.

6. The receiver circuitry according to any one of the preceding claims, further comprising a comparator (170), wherein the comparator (170) is connected to a node (120) of the power receiver (110) for comparing the amount of energy built up in the power receiver (110) to the energy threshold value, wherein the comparator (170) is configured to provide input to the control unit (150) in dependence of the comparing of the amount of energy built up in the power receiver (110) to the energy threshold value.

7. The receiver circuitry according to claim 6, wherein the comparator (170) is configured to compare a power receiver voltage at the node (120) of the power receiver (110) to a threshold voltage during a negative part of the power receiver voltage for providing input to the control unit (150) for switching the state of the first switch (126) and the state of the second switch (128) during a following positive part of the power receiver voltage.

8. The receiver circuitry according to any one of the preceding claims, further comprising a counter unit (162) configured to count a number of resonance cycles of the resonant circuit during build-up of energy.

9. The receiver circuitry according to any one of the preceding claims, wherein the control unit (150) is configured to receive the cycle threshold value, wherein the cycle threshold value is based on a quality factor of the resonant circuit (112).

10. The receiver circuitry according to any one of the preceding claims, wherein the second switch (128) comprises a transistor, wherein the second switch (128) is configured to be controlled between the open state and the closed state based on a signal to a gate of the transistor.

11. The receiver circuitry according to any one of the preceding claims, wherein the control unit (150) is configured to set the state of the first switch (126) to the open state in dependence of a voltage of the load reaching a target level.

12. An implant device (200) comprising the receiver circuitry (100) according to any one of the preceding claims, wherein the implant device (200) is configured to be implanted in a body and configured to receive power through wireless power transfer to the receiver circuitry (100).

13. A method for receiving power through wireless power transfer, said method comprising:

receiving (302), by a power receiver comprising a resonant circuit, an alternating current, AC, wireless power signal and storing energy through resonance by a first switch in the resonant circuit being closed for build-up of energy in the resonant circuit;
determining (304) a first condition based on a relation of a number of resonance cycles to a cycle threshold value and determining a second condition based on a relation of an amount of energy built up in the power receiver to an energy threshold value;
upon determining that the first condition or the second condition is met, controlling (306) switching of the first switch to an open state for forcing output of a current from an output node of the power receiver, and controlling switching of a second switch to a closed state for connecting the power receiver to a load.

Fig. 1

Fig. 2

*Fig. 3*

Receiving AC wireless
power signal and store
energy through resonance — 302

Determining first condition
based on relation between
number of resonance cycles and cycle
threshold value and determining
second condition based on
relation between amount of
energy built up and energy
threshold value — 304

First condition or
second condition met?

Yes

Controlling switching of first switch
to open state for forcing output
of current from power receiver
and controlling switching of second
switch to closed state for connecting
power receiver to load — 306

*Fig. 4*

| | Europäisches Patentamt European Patent Office Office européen des brevets | **EUROPEAN SEARCH REPORT** | **Application Number** EP 24 22 2093 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/334379 A1 (LEE SANG HAN [KR] ET AL) 31 October 2019 (2019-10-31) | 1,3-13 | INV. H02J7/00 |
| Y | * paragraphs [0035] - [0064]; figures 1-5 * | 2 | H02J50/12 |
| | ----- | | |
| X | US 2015/340873 A1 (LEE JAECHUN [KR] ET AL) 26 November 2015 (2015-11-26) * paragraphs [0090] - [0107], [0136]; figures 11-14,20-22 * | 1,13 | |
| | ----- | | |
| Y | US 2024/396375 A1 (NAGAI TAKAHIRO [JP] ET AL) 28 November 2024 (2024-11-28) * figure 5 * | 2 | |
| | ----- | | |
| A | EP 2 575 083 A1 (SAMSUNG ELECTRONICS CO LTD [KR]) 3 April 2013 (2013-04-03) * paragraphs [0036] - [0043]; figure 1 * | 1-13 | |
| | ----- | | |

**TECHNICAL FIELDS SEARCHED (IPC)**

H02J

The present search report has been drawn up for all claims

| Place of search | Date of completion of the search | Examiner |
|---|---|---|
| Munich | 5 May 2025 | Tchegho Kamdem, A |

## ANNEX TO THE EUROPEAN SEARCH REPORT
## ON EUROPEAN PATENT APPLICATION NO.

EP 24 22 2093

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2025

| Patent document cited in search report | | | Publication date | Patent family member(s) | | Publication date |
|---|---|---|---|---|---|---|
| US 2019334379 | A1 | | 31-10-2019 | KR | 20190125773 A | 07-11-2019 |
| | | | | US | 2019334379 A1 | 31-10-2019 |
| US 2015340873 | A1 | | 26-11-2015 | KR | 20150134107 A | 01-12-2015 |
| | | | | US | 2015340873 A1 | 26-11-2015 |
| US 2024396375 | A1 | | 28-11-2024 | JP | WO2023149316 A1 | 10-08-2023 |
| | | | | US | 2024396375 A1 | 28-11-2024 |
| | | | | WO | 2023149316 A1 | 10-08-2023 |
| EP 2575083 | A1 | | 03-04-2013 | CN | 103023537 A | 03-04-2013 |
| | | | | EP | 2575083 A1 | 03-04-2013 |
| | | | | JP | 5976481 B2 | 23-08-2016 |
| | | | | JP | 2013074794 A | 22-04-2013 |
| | | | | KR | 20130033704 A | 04-04-2013 |
| | | | | US | 2013080091 A1 | 28-03-2013 |

EPO FORM P0459

For more details about this annex : see Official Journal of the European Patent Office, No. 12/82